## Europäisches Patentamt
## European Patent Office
## Office européen des brevets

(11) Veröffentlichungsnummer: **0 428 636 B1**

# EUROPÄISCHE PATENTSCHRIFT

(45) Veröffentlichungstag der Patentschrift: **22.03.95**

(51) Int. Cl.6: **A61K 38/20**

(21) Anmeldenummer: **90904760.7**

(22) Anmeldetag: **28.03.90**

(86) Internationale Anmeldenummer:
**PCT/DE90/00248**

(87) Internationale Veröffentlichungsnummer:
**WO 90/11083 (04.10.90 90/23)**

Die Akte enthält technische Angaben, die nach
dem Eingang der Anmeldung eingereicht wurden
und die nicht in dieser Patentschrift enthalten sind.

(54) **VERWENDUNG VON INTERLEUKIN-2 ZUR BEHANDLUNG DER ENDOTOXINÄMIE.**

(30) Priorität: **28.03.89 DE 3910011**

(43) Veröffentlichungstag der Anmeldung:
**29.05.91 Patentblatt 91/22**

(45) Bekanntmachung des Hinweises auf die
Patenterteilung:
**22.03.95 Patentblatt 95/12**

(84) Benannte Vertragsstaaten:
**AT BE CH DE DK FR GB IT LI NL SE**

(56) Entgegenhaltungen:
**EP-A- 232 233**

**J. Clin. Invest. vol. 79, June 1979, The American Society for ClinicalInvestigation, Inc., C. Weyand et al.: "Administration in Vivo ofrecombinantinterleukin 2 protects mice against septic death", pages 1756-1763**

(73) Patentinhaber: **NITSCHE, Dietrich**
**Bismarckallee 2**
**D-24105 Kiel (DE)**

(72) Erfinder: **NITSCHE, Dietrich**
**Bismarckallee 2**
**D-24105 Kiel (DE)**

(74) Vertreter: **Tönnies, Jan G., Dipl.-Ing.**
**Niemannsweg 133**
**D-24105 Kiel (DE)**

Anaesthesia and Intensive Care, volume 17, no. 1, February 1989, J.G. Brock-Utne et al.: "Endotoxins and antiendoxins (their relevance to the anaesthetistand the intensive care specialist)"pages 49-55

FASEB, Meeting 19-23 March 1989, volum 3 no. 3, 1989, B. Crowley et al.: "Anti-endotoxin therapy-effect on survival, immune function and serum endotoxinlevels in a burn-sepsis model", page A917, abstract 4012.

Ann. Surg. volume 200, no. 3, 1984, J.J. Wood et al.: "Inadequate interleukin 2production. A fundamental immunological deficiency in patients with majorburns". pages 311-320.

The Lancet, volume 2, no. 8602, 9 July 1988, Y. Muto et al.: "Enhanced tumournecrosis factor and interleukin-1 in fulminant hepatic failure pages 72-74

**Beschreibung**

Die Erfindung betrifft die Verwendung von Interleukin-2 zur Zusatzbehandlung von krankhaften Zuständen, die mit einer Endotoxinämie einhergehen, mit dem Ziel, die Elimination von Endotoxinen aus der Blutbahn zu verbessern, die bei Gabe von bakteriziden Antibiotika verstärkt in das Blut übertreten können.

Interleukin-2(IL-2) ist ein Glykoprotein mit einem durchschnittlichen Molekulargewicht von 15 000 D. Es wird im menschlichen und tierischen Organismus, in geringen Mengen, vorwiegend von den T-Lymphocyten gebildet. IL-2 ist ein Mediator mit der Aufgabe, die Immunantwort bestimmter Zellen auf ein in den Organismus eingedrungenes Antigen zu regulieren. Das Interleukin-2 kann seine biologische Wirkung jedoch nur dann entfalten, wenn auf den entsprechenden Zielzellen genügend Rezeptoren für IL-2 vorhanden sind.

Aufgrund seiner biologischen Funktion als Regulator der Immunantwort wurden Überlegungen angestellt, IL-2 für die Therapie von Immunregulationsstörungen einzusetzen. Hierbei geht man davon aus, daß IL-2 in der Lage ist, die zellvermittelte Immunantwort zu verbessern. Bisher wurde Il-2 vorwiegend für die Zusatzbehandlung bösartiger Tumoren eingesetzt. Es wurde versucht. Tumorerkrankungen [ Lotze M.T. et al. (1986): High-Dose Recombinant Interleukin-2 in the Treatment of Patients with Disseminated Cancer: JAMA 256: 3117 - 3124 ] und Erkrankungen durch AIDS [ Vaith P. et al.(1985): In-vitro- und in-vivo-Studien mit Interleukin-2 und verschiedenen Immunstimulanzien bei einer Patientin mit AIDS; Immun.Infekt. 13: 51 -63 ] durch die Gabe von Interleukin-2 positiv zu beeinflussen.

Ein weiterer Anwendungsbereich für Il-2 wird in seiner Verwendung zur Prophylaxe oder Therapie von Infektionen gesehen, wobei Il-2 entweder allein oder in Kombination mit anderen Lymphokinen, wie z.B. mit dem Tumor-Nekrose-Faktor und/oder Interferon appliziert werden kann [ EP 0 242 233 A2 ]. Das Zielkriterium bei der Anwendung von Interleukin-2 zur Behandlung von Infektionen ist eine Verbesserung der unspezifischen zellulären und der spezifischen humoralen Abwehr. Da bei schweren Infektionen eine Herabsetzung der Il-2-Synthese zu einer Störung der Immunantwort führt und damit Bakterien leichter in den Organismus eindringen können, bietet es sich an, die unspezifische zelluläre Abwehr durch die Makrophagen durch die Substitution von IL-2 zu stimulieren. Wie experimentelle Untersuchungen gezeigt haben, gelang es durch die Gabe von Il-2, wobei rIl-2 verwendet wurde, die Letalität bei Tieren zu verbessern, denen eine Bakteriendosis in die Bauchhöhle appliziert wurde, die für die unbehandelte Kontrollgruppe letal war, ohne daß zusätzlich ein Antibiotikum gegeben wurde [ EP 0 242 233 A2; Chong KT; Prophylactic administration of Interleukin-2 protects mice from lethal challenge with gram-negative bacteria INFECT IMMUN 1987; 55: 668- 673; Gough DR. et al. Recombinant interleukin-2(rIL-2) improves immune response and host resistance to septic challenge in thermally injured mice. SURGERY 1988;104: 292 - 299 Maddaus MA, Simmons RL; Intraperitoneal administration of recombinant Interleukin-2 protects against lethal i.p. gram-negative sepsis by induction of a neutrophil influx. Surg Forum 1988; 39:105-106 ].

Die in EP 0 242 233 A2 sowie in den Druckschriften dargelegten Ergebnisse legen die Verwendung von Il-2 als Ersatz für die Gabe eines Antibiotikums nahe, da man durch die Applikation von Il-2 die Möglichkeit erhält, bakterielle Infektionen ohne die zusätzliche Gabe von Antibiotika zu behandeln. Die Experimente zeigen klar, daß der protektive Effekt durch die Applikation von Il-2 ausschließlich über einen verstärkten Einstrom der neutrophilen Granulocyten [ Maddaus MA, Simmons RL; 1988 ] sowie durch eine lokale Stimulation der Makrophagen [ Chong KT 1987 ] erreicht wurde. Hierauf weisen die folgenden zwei Beobachtungen [ Chong KT; Prophylactic administration of Interleukin-2 protects mice from lethal challenge with gram-negative bacteria INFECT IMMUN 1987; 55: 668- 673 ] eindeutig hin:

a) durch die Il-2 Gabe konnte ein protektiver Effekt nur dann erreicht werden, wenn Interleukin-2 auf die gleiche Weise appliziert wurde, wie die Bakterien, d.h. bei intraperitonealer Gabe der Bakterien nur dann, wenn rIl-2 ebenfalls intraperitoneal gegeben wurde. Bei intravenöser Gabe von Il-2 und gleichzeitger intraperitonealer Bakteriengabe fehlte der protektive Effekt des rIl-2 jedoch gänzlich.

b) bei Vorbehandlung mit Carrageenan, einer hochmolekularen Polygalaktoseverbindung, die auf die Makrophagen toxisch wirkt, wurde durch die Il-2 Applikation bei bakteriellen Infektionen kein protektiver Effekt mehr erreicht.

Weyand et al.(1987) nutzten die stimulierende Wirkung von Il-2 auf die Antikörperproduktion für die aktive Immunisierung gegen Endotoxin, indem sie Mäusen die E.coli J5-Mutante, die allgemein als Antigen für die Bildung von Antkörpern gegen das Endotoxin verwendet wird, zusammen mit rIl-2 verabreichten. Es wurde auf diese Weise ein Anstieg der Antikörper gegen Endotoxin in der IgM-Fraktion erreicht. In weiteren Tierexperimenten, bei denen dieses Serum zur passiven Immunisierung verwendet wurde, konnte durch das Serum ein Schutzeffekt vor septischen Komplikationen erreicht werden [ Weyand C, Goronzy J, Fathman CG, O'Hanley P; Administration in vivo of recombinant interleukin 2 protects mice against septic death. J Clin Invest 1987; 79:1756-1763 ]. Dieser Schutzeffekt war nur mit der IgM-Fraktion übertragbar. Die Ergebnisse dieser experimentellen Untersuchungen, legen die prophylaktische Gabe von rII-2 zum Zwecke der verbesserten Immunisierung von Risikopatienten durch eine Stimulation der körpereignen Produktion spezifischer IgM-Antikörper nahe, mit dem Ziel, dadurch die Prognose von Risikopatienten bei einer in der Folgezeit evtl. auftretenden septischen Komplikation zu verbessern.

EP 0 242 233 A2 sowie die hier zitierten Druckschriften legen die Verwendung von Interleukin-2 als Alternative zur Gabe eines Antibiotikums nahe, mit dem Ziel, bei bakteriellen Infektionen, eine Reduzierung der Bakterienzahlen durch eine Steigerung der Makrophagenaktivität zu erreichen. Die gleichzeitige Gabe eines Antibiotikums wird in diesen Schriften nicht empfohlen. Eine wesentlich radikalere und damit auch therapeutisch effizientere Reduktion der Bakterien ist jedoch nur durch die Gabe eines Antibiotikums zu erwarten. Daher bietet es sich an, für den in EP 0 242 233 A2 sowie in den zitierten Druckschriften nahegelegten Anwendungsbereich für das Interleukin-2, besonders dann, wenn ausgedehntere bakteriellen Infektionen vorliegen, anstelle von Il-2 vorzugsweise ein Antbiotikum als wirksamere Alternative zu verwenden.

Hinweise dafür, daß die Verbesserung der Letalität durch eine Steigerung der Endotoxinelimination erreicht wird, ergeben sich weder aus EP 0 242 233 A2 noch aus den zitierten Druckschriften. Durch die Phagocytose-Steigerung wird die Bakterienzahl vermindert, ohne daß dadurch der Bakterienzerfall und damit die Freisetzung von Endotoxin gesteigert wird, was bei Gabe von Antibiotika regelmäßig der Fall ist.

Für therapeutische Zwecke stehen natürliches Intereleukin-2(nII-2) sowie rekombinantes Interleukin-2(rII-2) zur Verfügung. Die Herstellung von natürlichem Interleukin-2 erfolgt aus humanen, peripheren Lymphocyten, wobei vorwiegend der Buffy-coat verwendet wird, der bei der Herstellung von Blutkonserven anfällt. Hierzu werden aus dem Buffy-coat die Lymphocyten isoliert und in Zellkulturen mit Lektin ( Phytohämagglutinin ) zur Synthese von Interleukin-2 stimuliert. Anschließend erfolgt die Abtrennung der Zellen von der Kulturflüssigkeit. Diese Ausgangsmaterial wird für die Konzentrierung des IL-2 verwendet. Für die Therapie liegt das natürliche Interleukin-2 vorwiegend in der verträglichen, glykolysierten Form vor. Als Stabilisator wird Albumin benötigt.

Rekombiniertes Interleukin-2 (rII-2) wird durch die rekombinante DNA-Technik aus E. coli- Bakterien sowie aus Hefe gewonnen. Das rekombinierte Interleukin-2 liegt im Gegensatz zu dem natürlich vorkommenden IL-2 in der unglykolysierten Form vor. Die Aminosäuresequenz des rII-2 unterscheidet sich von der des nII-2 dadurch, daß das endständige Alanin fehlt und daß das Cystein in Position 125 durch Serin ersetzt ist. Bei der Behandlung der Patienten mit rekombiniertem IL-2 wurden sehr starke Nebenwirkungen beobachtet ( LOTZE M.T. et al (1986): Clinical effects and toxicity of recombinant Interleukin-2 in patients with cancer. CANCER 58: 2764 -2772 ). Diese Nebenwirkungen werden vorwiegend auf die fehlende Glykolysierung sowie zum Teil auch auf die Stabilisatoren zurückgeführt, die zugesetzt werden müssen.

Endotoxin ist ein Zellwandbestandteil der gramnegativen Bakterien, der erst bei dem Bakterienzerfall freigesetzt wird. Es ist ein Makromolekül mit einem Molekulargewicht bis zu $1 \times 10^6$ Dalton, das sich vorwiegend aus Zuckerverbindungen und Fettsäuren zusammensetzt. Daneben kann es noch Proteinreste angelagert haben, die von der Bakterienwand stammen. Das Endotoxinmolekül ist aus drei strukturell und immunbiologisch unterschiedlichen Subregionen aufgebaut:

1.) Die Subregion 1, die O-spezifische Kette, besteht aus mehreren, sich wiederholenden Oligosaccharideinheiten, die jeweils von maximal 5 Neutralzuckern gebildet werden. Die Anzahl der Oligosaccharide ist von der Bakterienart abhängig; z.B. hat das hier untersuchte Endotoxin von S. abortus equi in dieser Region 8 Oligosaccharid-Einheiten.

2.) Die Subregion 2, das Kernoligosaccharid besteht u.a. aus N-Acetylglucosamin, Glucose, Galactose, Heptose und 2-Keto-3-Desoxyoctonsäure.

3.) Die Subregion 3, das Lipid-A ( MG 2000 Dalton ) besteht aus einem phosphorylierten D-Glukosamin-Disaccharid, an das mehrere - ca. 7 - langkettige Fettsäuren in Amid- und Esterbindung geknüpft sind. Der Träger der toxischen Eigenschaften ist das Lipd-A, wobei die toxische Wirkung von einigen Fettsäureresten in dieser Region ausgeht.

Im Organismus bewirkt Endotoxin einerseits die Freisetzung schädigender Mediatoren, andererseits führt es zur Störung des Energiestoffwechsels der Zelle, woraus ein Zelluntergang und - bei höherer Endotoxindosis und/oder längerer Endotoxinämiedauer - schließlich ein Organversagen resultieren kann. Das klinische Bild der gramnegativen Sepsis korreliert in den meisten Fällen mit dem Verlauf und der Höhe der Endotoxinkonzentration im Blut [ Nitsche D, Kriewitz M, Rossberg A, Hamelmann H.: The quantitative determination of endotoxin in plasma samples of septic patients with peritonitits using the chromogenic substrate and its correlation with the clinical course of peritonitis. In: Watson SW, Levin J, Novitsky TJ, eds. Detection of bacterial endotoxins with the Limulus amebocyte lysate test. New York: Alan R Liss, 1987; 417-429 ]. Bei Patienten, bei denen über mehrere Tage Endotoxin im Blut nachgewiesen wurde, war es in allen beobachteten Fällen zum Tod durch Organversagen gekommen.

4

Bei folgenden krankhaften Zuständen kann Endotoxin vermehrt im Blut auftreten:

a) bei einem vermehrten Übertritt aus dem Intestinaltrakt, aufgrund von Permeabilitätsstörungen oder aufgrund einer vermehrten Freisetzung als Folge einer enteralen Antibiotikatherapie;

b) bei einer Störung der Elimination über die Leber, z.B. bei Leberparenchymschäden oder nach Leberteilresektion.

c) bei Übertritt von einem infizierten Herd, der mit gramnegativen Bakterien besiedelt ist.

zu a) Endotoxin tritt physiologischerweise auch beim Gesunden aus dem Darm in die Blutbahn über. Die Elimination des Endotoxins aus dem Pfortaderblut erfolgt vorwiegend über die Leber. Zum Schutz vor einer Organschädigung durch Endotoxin hat das Plasma des Gesunden die Fähigkeit, das ständig aus dem Darm übertretende Endotoxin zu inaktivieren. Der Mechanismus, mit dem dieses erfolgt, ist noch nicht aufgeklärt. Störungen der Permeabilität im Bereich des Intestinaltraktes, wie sie beispielsweise bei schockbedingten Mikrozirkulationsstörungen, sowie im Rahmen von entzündlichen Darmerkrankungen und bei Immunsuppression mit Ciclosporin A auftreten, können zu einem gesteigerten Übertritt von Endotoxin aus dem Darmlumen in die Blutbahn führen. Ein vermehrter Übertritt von Endotoxin aus dem Intestinaltrakt in die Blutbahn ist auch dann zu beobachten, wenn es im Rahmen einer enteralen Antibiotikagabe zu einer gesteigerten Endotoxinfreisetzung im Darm kommt [Nitsche D, Stehle M, Hamelmann H. Der Einfluß der Immunsuppresion mit Ciclosporin auf die enterogene Endotoxinämie: Tierexperimentelle Untersuchungen. Langenbecks Archiv für Chirurgie, im Druck].

zu b) Bei Patienten, bei denen eine Störung der exkretorischen Funktion der Leber vorliegt, wie z.B. bei Patienten mit einem Verschlußikterus, kann biologisch aktives Endotoxin im Plasma nachgewiesen werden, dessen Konzentration mit der Höhe des Bilirubingehaltes im Blut korreliert. Aufgrund der gestörten Elimination über die Leber hat die Behandlung unspezifischer Infektionen mit Antibiotika bei diesen Patienten einen starken Endotoxinanstieg im Blut zur Folge, was vorwiegend auf eine verstärkte Endotoxinfreisetzung im Intestinaltrakt zurückzuführen ist [ Nitsche et al.: Der Plasmaendotoxinspiegel beim Verschlußikterus. Publ. in Vorbereitung ].

zu c) Bei ausgedehnten, gramnegativen Infektionen, wie z.B. bei der Peritonitis kann es zu einer starken Endotoxinfreisetzung und damit zu einem vermehrten Endotoxinübertritt von dem septischen Herd in die Blutbahn kommen. Wenn bei diesen Infektionen zusätzlich ein Antibiotikum gegeben wird, dann nimmt die Endotoxinfreisetzung sehr stark zu. Bei intraabdominellen Infektionen kann man bereits 30 Minuten nach Gabe eines Antibiotikums einen exponentiellen Anstieg der Endotoxinaktvität im Plasma beobachten [ Goto H, Nakamura S. Liberation of endotoxin from Escherichia coli by addition of antibiotics. Jap J Exper Med 1980;50: 35 - 43; Shenep JL, Mogan KA. Kinetics of endotoxin release during antibiotic therapy for experimental gram-negative bacterial sepsis. J Infect Dis 1984;150:380 -388; Cohen J, McConnell JS. Antibiotic-induced endotoxin release. Lancet 1985;1069; Van Baalen A, Nitsche D, Hamelmann H. Endotoxin release by antibiotics in peritonitis. Langenbecks Arch Chir 1989;Suppl; 303 - 306 ]. Bei einer derartig massiven Endotoxinfreisetzung, wie sie als Folge einer Antibiotikatherapie immer auftreten kann, ist der Organismus besonders im Rahmen der bei ausgedehnten gramnegativen Infektionen zu erwartenden Verschlechterung der Organfunktion nicht mehr in der Lage, die in die Blutbahn übergetretenen Endotoxine ausreichend schnell wieder zu eliminieren. Im Tierexperiment können sämtliche Symptome und Organveränderungen eines septischen Schocks ausgelöst werden, wenn man die Tiere mit einer entsprechend großen Menge gramnegativer Bakterien infiziert und anschließend ein bakterizides Antibiotikum verabreicht.

Bei einem massiven Endotoxineinstrom, wie er z.B. im Rahmen einer Eliminationsstörung sowie im Rahmen einer Antibiotikatherapie auftreten kann, erschöpft sich die Fähigkeit des Plasmas zur Inaktivierung des Endotoxins rasch, sodaß vermehrt biologisch aktives Endotoxin im Plasma auftritt, welches dann zu dem klinischen Bild der Endotoxinämie führt. Die Endotoxinämie kann - je nach Dosis und Dauer - schließlich ein irreversibles Organversagen verursachen. Andererseits gehört jedoch die Gabe von Antibiotika zu dem unverzichtbaren, therapeutischen Standard bei bakteriellen Infektionen. Aus diesem Grund sind bei allen Erkrankungen, bei denen ein vermehrter Endotoxinübertritt von einem septischen Herd oder aus dem Darmlumen in das Blut zu erwarten ist, besonders im Rahmen einer notwendigen Antibiotikatherapie, sowie bei Erkrankungen, bei denen die physiologische Endotoxinelimination über die Leber gestört ist, zusätzliche therapeutische Maßnahmen wünschenswert, mit denen es gelingt, die Endotoxinaktivität Im Blut herabzusetzen.

Als therapeutische Maßnahmen zur Herabsetzung der Endotoxinaktivität Im Plasma kommen die intravenöse Applikation von Hyperimmungiobulin-Präparationenmit einem hohen Gehalt an Lipid-A-Antikörpern sowie die intravenöse Gabe anderer Plasma-Proteine - insbesondere aus der Gruppe der Siderophiline - in Frage, die in der Lage sind, Endotoxin zu inaktivieren. Eine weitere Möglichkeit besteht darin, durch entsprechende therapeutische Schritte den physiologischen Endotoxineinstrom aus dem Intestinaltrakt herabzusetzen.

Eine zusätzliche Maßnahme zur Behandlung der Endotoxinämie ist in einer Stimulation der Endotoxinelimination des Organismus durch Gabe eines geeigneten Zusatztherapeutikums zu sehen, welches man dann z.B. auch im Rahmen einer geplanten Therapie mit Antibiotika verabreichen kann.

Der Erfindung liegt die Aufgabe zugrunde, eine Mittel zu finden, welches in der Lage ist, die natürliche Elimination des Endotoxins aus der Blutbahn durch den Organismus zu steigern, und zwar in den Fällen, in denen ein vermehrter Endotoxinübertritt aus dem Intestinaltrakt oder von einem septischen Herd nach Gabe eines Antibiotikums zu erwarten ist, sowie in den Fällen, in denen die Endotoxinelimination durch eine krankhafte Störung der Organfunktion vermindert ist. Dabei soll das Mittel auch in der Lage sein, die Clearance der bei einer erforderlichen Therapie gramnegativer Infektionen mit Antibiotika verstärkt freigesetzten Endotoxine derart zu steigern, daß die zusätzliche Belastung des Organismus durch die hierbei zwangsläufig auftretende, verstärkte Endotoxineinschwemmung herabgesetzt wird.

Erfindungsgemäß wird diese Aufgabe gelöst durch die Verwendung von Interleukin-2. Dieses kann subcutan, intramuskulär, sowie intravenös und intraperitoneal appliziert werden. Es eignet sich besonders zur Zusatzbehandlung bei der Gabe bakterizider Antibiotika. Dabei kann es sich um rekombiniertes Interleukin-2 (rIL-2) und natürliches Interleukin-2 (nIl-2) handeln.

Zur Prüfung der Frage, inwieweit sich Interleukin-2 für die Verbesserung der Endotoxinelimination aus dem Organismus eignet, wurden tierexperimentelle Untersuchungen an Ratten durchgeführt.

Grundbedingung für eine Stimulation der Zellen durch IL-2 ist eine ausreichende Dichte von IL-2-Receptoren auf der Oberfläche der Zielzellen. Diese Voraussetzung ist besonders bei immunsupprimierten Patienten, sowie nach schweren Traumen, nach Operationen und bei ausgedehnten Infektionen gewährleistet, da es hier zu einer Herabsetzung der Il-2 Produktion kommt, was mit einer vermehrten Ausbildung von Il-2-Receptoren einhergeht. Um im Tierexperiment mögliche Effekte des IL-2 reproduzierbar zu erkennen, wurden Bedingungen geschaffen, bei denen eine vermehrte Exprimierung von IL-2-Receptoren gewährleistet ist. Hierzu wurde bei Wistar-Ratten (250 - 350 g) die Interleukin-2-Synthese durch eine viertägige Vorbehandlung mit Cyclosporin A ( 15 mg/kg pro Tag ) unterdrückt.

Für die tierexperimentellen Untersuchungen wurde natürliches, humanes Interleukin-2 verwendet, welches den durch Immunsuppression entsprechend vorbehandelten Ratten intraperitoneal, sowie subcutan und intravenös verabreicht wurde. Die Kontrolltiere erhielten NaCl anstelle von Il-2. Für das natürliche Interleukin-2 wurden folgende Dosierungen gewählt: $1 \times 10^5$ U pro kg Körpergewicht; $3.5 \times 10^5$ U pro kg Körpergewicht; $1,75 \times 10^6$ U pro kg Körpergewicht, $3,5 \times 10^6$ U pro kg Körpergewicht und $1,75 \times 10^7$ U pro kg Körpergewicht.

Verschiedene Zeiten und zwar 0 bis 24 Stunden nach Il-2-Gabe wurden den Tieren in Narkose Coli-Bakterien in einer Dosierung von $2,2 \times 10^6$ cfu/ml pro kg Körpergewicht intraperitoneal appliziert. Zur Erzeugung einer starken Endotoxinfreisetzung, verbunden mit einem massiven Endotoxinübertritt aus der Bauchhöhle in das Blut, wurde den Tieren eine Stunde nach der intraperitonealen Bakteriengabe ein bakterizides Antibiotikum ( Imipenem: 14 mg pro kg Körpergewicht ) i.v. verabreicht. Zur Bestimmung der Endotoxinkonzentration im Plasma sowie zur Bestimmung der Bakterienzahl im Blut wurde vom Versuchsbeginn an, regelmäßig alle 30 Minuten, bis zum Versuchsende Blut ( 0,25 ml ) aus der vena jugularis entnommen. Versuchsende war 5 Stunden nach Gabe des Antibiotikums, d.h. die Gesamtversuchsdauer betrug jeweils 6 Stunden.

Unmittelbar nach der Abnahme aus der Vene wurden für die Endotoxinbestimmung 200 µl Blut sofort bei 4°C abzentrifugiert ( 3 000 x g ), um die im Blut evtl. vorhandenen Bakterien abzutrennen. Anschließend wurden 100 µl der Plasmaprobe ( Heparin-Plasma) sofort 1:40 in 0.9% NaCl verdünnt und 5 Minuten bei 80 °C erhitzt. Danach wurde die Probe rasch abgekühlt und bei -80°C für die Endotoxinbestimmung aufbewahrt. Die quantitative Bestimmung der biologischen Aktivität des Endotoxins im Plasma erfolgte mit einer Modifikation des Limulus-Test, unter Verwendung eines chromogenen Substrates ( Nitsche et al. In: Watson, SW, Levin J, Novitsky TJ (eds) DETECTION OF BACTERIAL ENDOTOXINS WITH THE LIMULUS AMEBOCYTE LYSATE TEST. pp 417-429 ( 1987 )). Die untere Nachweisgrenze liegt bei dieser Modifikation des Limulus-Tests bei 2 EU/dl ( 1 EU/dl = 100 pg/dl EC-5 ). Die mit dem Limulus-Test erfaßte Endotoxinaktivität korreliert mit der biologischen Aktivität des Endotoxins ( Nitsche D, Flad HD und Blum B (1990) Endotoxin determination by the Limulus-Test and the biological activity of endotoxin. Does a correlation exist? Publikation in Vorbereitung ).

Ergebnisse: ( Abb.: 1; Tab.I )

Bereits 30 Minuten nach Bakteriengabe in die Bauchhöhle können im Blut Bakterien nachgewiesen werden. Bei den Tieren der Kontrollgruppe, die statt Il-2 nur NaCl erhalten hatten, steigt die Bakterienzahl im Blut signifikant höher an, als bei den Tieren, die vorher natürliches Interleukin-2 i.p. bekommen hatten.

Die i.v.-Gabe des bakteriziden Antibiotikums, die eine Stunde nach Bakteriengabe vorgenommen wurde, führt sowohl bei der NaCl- wie auch bei der Il-2 Gruppe zu einer raschen Abnahme der Bakterien in der Blutbahn. Gleichzeitig tritt bei den Tieren, die nicht mit nIL-2 vorbehandelt waren, bereits 30 Minuten nach Gabe des Antibiotikums ein starker Anstieg der Endotoxinaktivität im Plasma auf.

Im Gegensatz dazu kann bei den Tieren, die mit Il-2 behandelt waren, nur eine vergleichsweise geringere Zunahme der Endotoxinaktivität im Plasma nach Gabe des Antibiotikums gefunden werden. Bei diesen Tieren findet sich zwischen der verabreichten nIl-2 Dosis und der Endotoxinaktivität im Plasma eine Beziehung. Es zeigt sich, daß eine gewisse Il-2-Mindestdosis erforderlich ist, um durch die Zusatztherapie mit nIl-2 nach Gabe des Antibiotikums eine signifikant niedrigere Endotoxinaktivität im Plasma zu erreichen, als bei der NaCl-Kontrollgruppe.

a) Bei den Immunsupprimierten Tieren, welche die niedrigste nIl-2-Dosis von $1 \times 10^5$ U pro kg Körpergewicht bekommen hatten, fand sich nach Gabe des Antibiotikums im Vergleich zur NaCl-Kontrollgruppe kein statistisch signifikanter Unterschied zwischen den Werten für die Plasma-Endotoxin-aktivitäten beider Gruppen.

| Z E I T | Plasma-Endotoxinkonz. [EU/dl] | | | | | |
| | 0 | 1 h | 2 h | 3 h | 4 h | 5 h |
| --- | --- | --- | --- | --- | --- | --- |
| NaCl-Gruppe | 0 | 12 ± 5 | 486 ± 97 | 445 ± 45 | 409 ± 58 | 417 ± 61 |
| nIl-2 Gruppe | 0 | 6 ± 4 | 64 ± 16 | 70 ± 15 | 68 ± 14 | 72 ± 11 |
| | | n.s. | p< 0.001 | p< 0.001 | p< 0.001 | p< 0.001 |

Tab.I: Einfluß der Zusatztherapie mit nIl-2 auf die Plasmaendotoxin-aktivität bei einer durch Antibiotikagabe induzierten Endotoxinämie

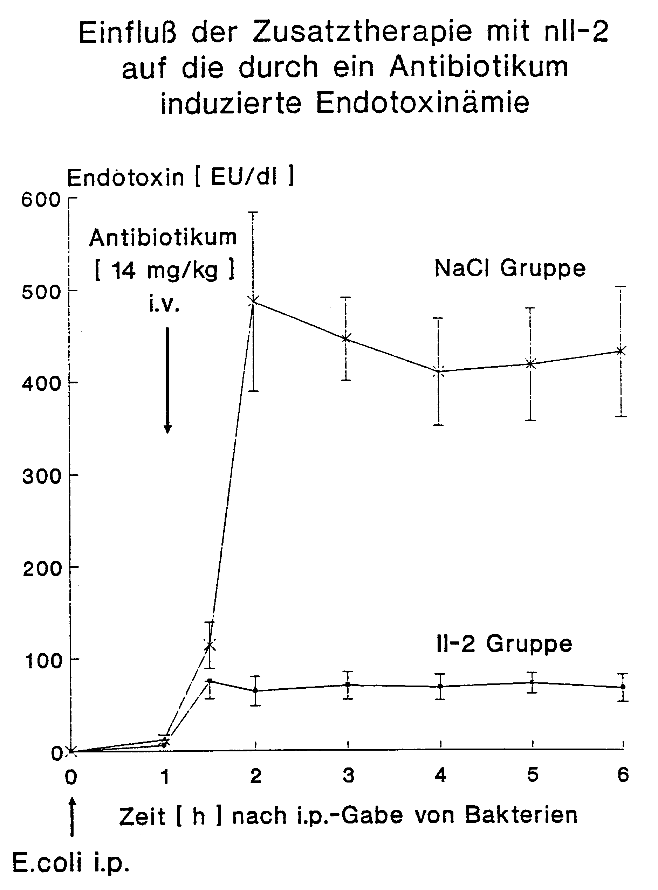

b) Bei den immunsupprimierten Tieren, welche eine Il-2-Dosis von $3.5 \times 10^5$ U pro kg Körpergewicht intraperitoeal oder subcutan bekommen hatten, unterschied sich die Endotoxinaktivität im Plasma signifikant von den Werten, die bei der NaCl-Kontrollgruppe gefunden wurden. Bei diesen Tieren war der Endotoxinanstieg im Plasma, nach Gabe des Antibiotikums, signifikant ( p < 0.001 ) niedriger als bei der NaCl-Kontrollgruppe ( Abb.1; Tab I ).

Bereits eine Stunde nach Gabe des Antibiotikums beträgt die Endotoxinaktivität im Plasma bei der Kontrollgruppe 486 ± 97 EU/dl ( n = 14 ) und ist damit ca. 86 % höher als bei den Tieren der nIl-2 Therapiegruppe, bei denen die Endotoxinaktivität zu diesem Zeitpunkt nur 64 ± 16 EU/dl ( n = 16 ) beträgt. Bei Versuchsende, d.h. 5 Stunden nach Gabe des Antibiotikums, ist die Plasma-Endotoxinaktivität bei der NaCl-Kontrollgruppe ca 83 % höher als bei den mit nIl-2 vorbehandelten Tieren ( Tab. I ).

c) Bei intraperitonealer oder auch intramuskulärer Gabe des nIL-2 in einer Dosierung von $1,75 \times 10^6$ bzw $3,5 \times 10^6$ U und $1,75 \times 10^7$ U pro kg Körpergewicht fanden sich ähnliche Werte für die Endotoxinaktivität im Plasma, wie bei einer Dosierung von $3,5 \times 10^5$ U pro kg Körpergewicht. Das bedeutet, daß der endotoxinsenkende Effekt des nIl-2, durch Stimulation der körpereignen Elimination, durch eine Erhöhung der Dosis über $3,5 \times 10^5$ U pro kg Körpergewicht nicht mehr signifikant verbessert werden kann.

d) Bei Intravenöser Gabe des nIL-2 in einer Dosierung von $3,5 \times 10^5$ U pro kg Körpergewicht konnte im Vergleich zur NaCl-Kontrollgruppe kein statistisch signifikanter Effekt auf die Endotoxinaktivität Im Plasma nachgewiesen werden. Erst bei einer Dosis von $1,75 \times 10^7$ U pro kg Körpergewicht wurde bei intravenöser Gabe des natürlichen Interleukin-2 im Vergleich zur Kotrollgruppe ein statistisch signifikanter Effekt auf die Endotoxinaktivität im Plasma gefunden, der jedoch etwas geringer war, als bei intraperitonealer oder subcutaner Gabe. Bei i.v.-Gabe von Il-2 betrug die Senkung der Plasma-Endotoxinaktivität durch das Il-2 bei Versuchsende im Vergleich zur Kontrollgruppe 61 %.

Ein Effekt von nII-2 auf die Plasma-Endotoxinaktivität kann bereits eine Stunde nach Il-2 Gabe gefunden werden. Die stimulierende Wirkung der Il-2 Gabe auf die Endotoxinelimination hält nach einer einmaligen Il2-Applikation bis maximal 24 Stunden an. Bei Tieren, bei denen erst 36 Stunden nach Il-2-Gabe eine bakterielle Infektion mit anschließender Endotoxinämie induziert wurde, unterschieden sich die Endotoxinaktivitäten im Plasma während des gesamten Beobachtungszeitraumes nicht signifikant von den Werten, die bei der unbehandelten Kontrollgruppe gefunden wurden.

Zusammenfassende Beurteilung der Ergebnisse:

1. Interleukin-2 ist in der Lage, die physiologische Elimination des Endotoxins aus dem Organismus zu stimulieren und damit die Endotoxin-Clearance zu steigern.

2. Aufgrund seiner Eigenschaft, die Endotoxin-Clearance zu verbessern, eignet sich IL-2 bevorzugt als Zusatztherapeutikum für die Behandlung ausgedehnter, gramnegativer Infektionen zusammen mit Antibiotika, mit dem Ziel, die Gefahr der Schädigung des Organismus durch das Endotoxin herabzusetzen, welches durch die Antibiotikagabe sowohl von dem Infizierten Herd, wie auch aus dem Intestinaltrakt zwangsläufig verstärkt freigesetzt wird.

3. Die Gabe von Il-2 eignet sich auch für die Zusatzbehandlung zur Steigerung der Endotoxinelimination, besonders bei Patienten, bei denen die Endotoxin-Clearance z.B. durch eine Störung der Leberfunktion oder auch durch eine Leberteilresektion eingeschränkt ist.

4. Die Stimulation der Endotoxinelimination durch das Il-2 ist besonders dann sehr effektiv, wenn an den Zielzellen eine ausreichend hohe Dichte an Interleukin-2-Receptoren vorhanden ist. Da diese Situation besonders bei Patienten mit einer Immunsuppression sowie nach großen operativen Eingriffen, nach Traumen und bei ausgedehnten Infektionen, insbesondere auch bei der Sepsis, zu erwarten ist, eignet sich IL-2 bevorzugt in diesen Fällen als Zusatztherapeutikum zur Stimulation der Endotoxinelimination.

DIE ERFINDUNG WIRD DURCH FOLGENDE BEISPIELE ERLÄUTERT:

1. Zur Klärung der Frage, ob bei eingeschränkter Leberfunktion eine Steigerung der Endotoxinelimination durch die Zusatztherapie mit Il-2 erreicht werden kann, wurden folgende tierexperimentelle Untersuchungen durchgeführt:

Bei Wistar-Ratten (250 - 350 g), bei denen die Interleukin-2-Synthese durch eine viertägige Vorbehandlung mit Cyclosporin A ( 15 mg/kg pro Tag ) gehemmt worden war, wurde zwei Tage vor Versuchsbeginn eine Leberteilresektion durchgeführt, wobei bei einer Gruppe ca. 50 % der Leber reseziert wurde. Bei einer weiteren Gruppe der Tiere wurden 90 % der Leber reseziert. Bei einer Kontrollgruppe war keine Leberresektion durchgeführt worden.

Zwei Stunden vor Versuchsbeginn erhielten die Tiere der Therapiegruppe jeweils rIl-2 In einer Dosis von $3,5 \times 10^5$ U pro kg Körpergewicht subcutan verabreicht. Die Tiere der Kontrollgruppen erhielten stattdessen NaCl.

Entsprechend den Bedingungen, wie sie bei der Therapie von gramnegativen Infektionen mit Antibiotika zu finden sind, wurde der Endotoxinanstieg im Blut bei den Tieren dadurch induziert, daß zunächst Bakterien in die Bauchhöhle gegeben wurden und durch die anschließende intravenöse Gabe eines bakteriziden Antibiotikums ein rascher Zerfall dieser Bakterien ausgelöst wird. Zur Blutabnahme wurde den Tieren zunächst in Narkose ein Katheter in die rechte Halsvene (Vena Jugularis ) eingelegt. Anschließend wurden den Tieren Coli-Bakterien in einer Dosierung von $2,2 \times 10^6$ cfu/ml pro kg Körpergewicht intraperitoneal verabreicht. Zur Erzeugung einer Endotoxinämie wurde eine Stunde nach Bakteriengabe ein bakterizides Antibiotikum (IMIPENEM = Zienam[R] :14 mg pro kg Körpergewicht ) intravenös appliziert. Die Blutabnahmen aus der Vena jugularis zur Bestimmung der Endotoxinaktivität und der Bakterienzahl erfolgten vor, sowie - in 30 minütigem Abstand - nach der Bakteriengabe über insgesamt 5 Stunden. Versuchsende war 5 Stunden nach i.p. Gabe der Bakterien.

Bei sämtlichen Tieren, die mit rIl-2 vorbehandelt waren, d.h. auch bei den Tieren mit einer Leberteilresektion, waren die Bakterienzahlen im Blut zum Zeitpunkt der Gabe des Antibiotikums signifikant niedriger, als bei den unbehandelten Tieren der NaCl-Gruppe.

Der Verlauf der Endotoxinaktivität im Plasma, nach Gabe des Antibiotikums zeigte bei den verschiedenen Gruppen ein deutlich unterschiedliches Verhalten:

1.) bei den Tieren mit einer intakten Leber kam es nach Gabe des Antibiotikums bei der NaCl-Kontrollgruppe ( n = 12 ) zu einem starken Anstieg der Endotoxinaktivität im Plasma. Die Werte für die Endotoxinaktivität waren hier signifikant höher, als bei den Tieren, bei denen vor Gabe des Antibiotikums eine Zusatztherapie mit rIl-2 ( n = 12 ) durchgeführt wurde ( Tab.: 2 ).

2.) Bei den Tieren, bei denen eine Leberteilresektion durchgeführt worden war, findet man im Gegegensatz zu der nicht operierten Kontrollgruppe bereits bei Versuchsbeginn, d.h. vor Bakteriengabe eine leicht erhöhte

| ZEIT | PLASMA - ENDOTOXINKONZ. [ EU / dl ] | | | | | |
|------|------------------|------------|-----------|----------|----------|----------|
| | normale Leberfunktion | | Leberteilresektion | | | |
| | | | 50 % Resektion | | 90 % Resektion | |
| | NaCl | rIl2 | NaCl | rIl-2 | NaCl | rIl-2 |
| O h | 0 | 0 | 42 ± 8 | 25 ± 8 | 119 ± 9 | 124 ± 11 |
| 1 h | 11 ± 4 | 12 ± 5 | 85 ± 21 | 41 ± 18 | 166 ± 37 | 193 ± 32 |
| 2 h | 428 ± 72 | 74 ± 23 | 512 ± 61 | 263 ± 34 | 698 ± 58 | 712 ± 78 |
| 3 h | 402 ± 87 | 76 ± 18 | 543 ± 92 | 268 ± 21 | 813 ± 102 | 792 ± 91 |
| 4 h | 369 ± 68 | 72 ± 14 | 597 ± 79 | 318 ± 40 | 922 ± 97 | 938 ± 107 |
| 5 h | 398 ± 59 | 69 ± 12 | 581 ± 62 | 338 ± 47 | 941 ± 86 | 926 ± 94 |

Tab.II: Einfluß der Zusatztherapie mit rIl-2 auf die Plasmaendotoxin-aktivität bei einer durch Antibiotika induzierten Endotoxinämie bei unterschiedlicher Leberfunktion

Endotoxinaktivität im Plasma, als Hinweis für eine herabgesetzte Endotoxinelimination durch die Leber.

2a) Bei den Tieren, bei denen ca. 50 % der Leber reseziert war, wurde durch die Zusatzbehandlung mit rIl-2 ( n = 11 ) erreicht, daß die Endotoxinaktivität bei Versuchsbeginn signifikant ( p < 0,001 ) niedriger war, als bei der NaCl-Kontrollgruppe. Bei der Il-2 Gruppe war der Anstieg der Endotoxinaktivität im Plasma, nach Gabe des Antibiotikums, ca. 47 % geringer, als bei der unbehandelten Kontrollgruppe ( n = 10 ) ( Tab.: 2 ). Die Unterschiede waren bis zum Versuchsende statistisch signifikant.

2b) Bei den Tieren, bei denen nur noch eine Restleber mit ca. 10% des Ausgangsvolumens vorhanden war, wurde durch die Zusatzbehandlung mit rIL-2 ( n = 14 ), im Vergleich zur unbehandelten Kontrollgruppe ( n = 15 ), keine signifikante Herabsetzung der nach Gabe des Antibiotikums sehr stark ansteigenden Endotoxinaktivität im Plasma erreicht ( Tab.: 2 ). Bei diesen Tieren zeigte die Endotoxinaktivität bei Versuchsbeginn ebenfalls keinen Unterschied zwischen der

II-2 Gruppe und der Kontrollgruppe.

Die Ergebnisse zeigen:

1. Die Leber ist Zielorgan für die Stimulation der Endotoxinelimination durch II-2.

2. Wie die Endotoxinkonzentration der Tiere, bei denen 50% der Leber reseziert wurden, bei Versuchsbeginn zeigt, kann durch die II-2-Gabe auch die Elimination der im Intestinaltrakt freigesetzten und in das Pfortaderblut übertretenden Endotoxine verbessert werden. Bei den Tieren dieser Gruppe, die mit II-2 vorbehandelt wurden, ist die Endotoxinaktivität im Plasma bereits bei Versuchsbeginn signifikant niedriger, als bei der NaCl-Kontrollgruppe.

3. Die Steigerung der Phagocytoseleistung der Makrophagen, die durch die Gabe von II-2 zu erwarten ist, führt nicht zwangsläufug auch zu einer Abnahme der Endotoxinaktivität im Plasma, wie die Endotoxinverläufe der Tiere beweisen, bei denen ca. 90% der Leber reseziert wurden,

2. Bei Patienten, die an einer diffusen Peritonitis erkrankt sind, kommt es durch das Überwiegen der gramnegativen Bakterien zu einer ständigen Endotoxinfreisetzung in der Bauchhöhle, sodaß von hier bereits vermehrt Endotoxine in die Blutbahn übertreten, auch wenn noch nicht mit der Antibiotika-Behandlung begonnen wurde. Eine schonende Eliminierung der Bakterien aus der Bauchhöhle durch Steigerung der Phagocytoseleistung der Makrophagen ist therapeutisch nicht wirkungsvoll genug, da besonders bei einer diffusen Peritonitis, wegen der starken bakteriellen Besiedlung nicht zu erwarten ist, daß auf diesem Wege eine ausreichend große Zahl der Bakterien eliminiert werden kann, um so einen Hellungsprozeß einzuleiten.

Bei Patienten mit ausgedehnten bakteriellen Infektionen, z.B. mit einer diffusen Peritonitis, ist damit die Gabe eines bakteriziden Antibiotikums die Therapie der Wahl. Die Antibiotika führen jedoch zwangsläufig zur plötzlichen Freisetzung größerer Mengen von Endotoxin, wobei einerseits der entzündliche Herd, andererseits die Bakterien des Intestinaltraktes die Endotoxinquelle sind. Entsprechende Untersuchungen bei Patienten mit einer diffusen Peritonits haben einen signifikanten Anstieg der Endotoxinaktivität im Plasma nach Gabe eines Antibiotikums gezeigt ( Nitsche et al; Publikation in Vorbereitung ).

Eine Verbesserung des klinischen Verlaufs der Peritonitiserkrankung ist u.a. dann zu erwarten, wenn es in Ergänzung zur radikalen Beseitigung der Bakterien gelingt, die Gefahr durch die vermehrt freigesetzten Endotoxine bereits bei Beginn der Therapie mit einem Antibiotikum herabzusetzen. Die zusätzliche Gabe von II-2 zusammen mit dem Antibiotikum, zum Zweck der Steigerung der Endotoxinelimination durch die Leber, eröffnet hier eine wirksame, therapeutische Möglichkeit, um die nachteilige Wirkung der Antibiotika zu vermindern. Durch die Zusatzbehandlung mit II-2 gelingt es, die Aktivität der durch die Antibiotikagabe freigesetzten Endotoxine im Plasma, bei normaler Leberfunktlon um ca. 70% bis 80% zu senken. Durch die Kombinationsbehandlung von II-2 zusammen mit dem Antibiotikum erhält man die Möglichkeit, die Endotoxinaktivität im Plasma trotz der Durchführung einer wirkungsvollen Therapie mit Antibiotika soweit herabzusetzen, daß das Risiko der toxischen Organschädigung für den Patienten veringert wird.

## Patentansprüche

1. Verwendung von Interleukin-2 zur Herstellung eines Arzneimittels zur Förderung der Elimination von Endotoxinen aus der Blutbahn als Zusatzbehandlung bei Gabe von gegen gramnegative Bakterien wirkenden Antibiotika.

2. Verwendung nach Anspruch 1, dadurch gekennzeichnet, daß das Interleukin-2 ein natürliches Interleukin-2 ist.

## Claims

1. Use of Interleukin-2 for manufacture of a drug to support the elimination of endotoxins from the bloodstream as supplementary treatment following the administration of antibiotics, working as an antidote against gramnegative germs.

2. Use as to claim 1, characterized by the fact that Interleukin-2 is a natural Interleukin-2.

**Revendications**

1. L'utilisation de l'Interleukin-2 pour la production d'un médicament pour effectuer la promotion de l'élimination des endotoxines de la circulation du sang comme traitement supplémentaire suivant l'administration des antibiotiques agissants contre les bactéries gramnégatives.

2. Selon la revendication 1 l'utilisation est caracterisée par le fait que l'Interleukin-2 est un Interleukin-2 naturel.

# Einfluß der Zusatztherapie mit nIl-2 auf die durch ein Antibiotikum induzierte Endotoxinämie

Endotoxin [ EU/dl ]

Antibiotikum [ 14 mg/kg ] i.v.

NaCl Gruppe

Il-2 Gruppe

Zeit [ h ] nach i.p.-Gabe von Bakterien

E.coli i.p.